# EUROPEAN PATENT APPLICATION

(11) **EP 4 789 700 A2**
(43) Date of publication of application: **12.08.2026**
(21) Application number: 26188467.0
(22) Date of filing: 23.04.2021
(51) Int. Cl.: A61M 5/315

(54) **ELECTRONIC MODULE AND DRUG DELIVERY DEVICE**

(30) Priority: 23.04.2020 EP 20315209; 26.06.2020 EP 20315325
(62) Document number(s) of the earlier application(s) in accordance with Art. 76 EPC: 21720488.2 / 4 138 956
(71) Applicant: Sanofi, 75017 Paris (FR)
(72) Inventor: HARVEY-COOK, Adam Moyo, Billericay, CM12 OUD (GB); EARWAKER, Tom Alexander, Leamington Spa, CV31 3EU (GB); PLUMPTRE, David Aubrey, Droitwich Spa, WR9 7RQ (GB); GAZELEY, Oliver Charles, 4058 Basel (CH); VEASEY, Robert Frederick, Warwick, CV34 4AB (GB); MASON, Craig Ashley, Helsby, WA6 0BA (GB)
(74) Representative: Keil & Schaafhausen Patentanwälte PartGmbB

(57) **Abstract**

The present invention refers to an electronic module (11) for releasable attachment to a drug delivery device (1) and to such a drug delivery device. The module (11) may comprise at least one module lockout form (323) adapted for mating abutment with a corresponding device lockout form (21) of a dedicated a drug delivery device (1). Further, the module (11) may comprise at least one attachment element (324) for releasable attachment of the module (11) on a drug delivery device (1). Further, the module (11) may comprise at least one light pipe (325) for guiding a light beam from a light source (121) to a reflective surface (24) of the drug delivery device (1) and from said reflective surface (24) to a light detector sensor (122). Still further, the module (11) may comprise at least one light guide (321) for guiding a light beam from a light source (141) to a user feedback surface (322) of said component adapted to emit light. Further, the module (11) may comprise at least one elastically deformable switch arm (326). The component (320) may be a unitary component part injection molded from a polycarbonate material.

## Description

The present invention is generally directed to an electronic system, e.g. a module, for a drug delivery device, and more specifically to a component part of such a module having multiple functions. The present invention further relates to a drug delivery device, which preferably comprises such an electronic module.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for disposable and reusable devices.

For such devices the functionality of recording doses that are dialled and/or delivered from the pen may be of value to a wide variety of device users as a memory aid or to support detailed logging of dose history. Thus, drug delivery devices using electronics are becoming increasingly popular in the pharmaceutical industry as well as for users or patients.

For example, a drug delivery device is known from EP 2 814 545 A1 comprising an electronic clip-on module. The clip-on module comprises a battery which powers a processor and further components controlled by the processor, like light-sources, a photometer, an acoustic sensor, an acoustical signal generator and a wireless unit, like a Bluetooth^{®} transceiver configured to transmit and/or receive information to/from another device in a wireless fashion.

However, especially if the device is designed to be self-contained, that is to say without a connector for a connection to an electrical power source which is necessary to provide electrical power for the operation of the device, the management of the resources of a power supply integrated into the device is particularly important.

Unpublished EP 20315066.9 and EP 20315357.2 disclose advantageous embodiments of electronic systems for drug delivery devices with improved power management. These electronic systems comprise a switch assembly for activating/deactivating power consuming functions of the electronic systems. Reference is made to these two documents regarding disclosure of the working principle of the electronic system in conjunction with the drug delivery device.

A further example of an electronic module for use with a drug delivery device is described in WO 2019/101962 A1. For detection of a dialled or dispensed dose, this module comprises an encoder system with two optical sensors each consisting of a transmitting portion, e.g. an LED, and a corresponding receiving portion, e.g. photodiodes. Light, e.g. IR light, emitted by the transmitting portion of the sensor is guided through a light pipe which extends from a chassis portion of the module to the vicinity of a ring of teeth formed on a proximal end of a rotatable number sleeve of the drug delivery device. The light exits the light pipe at the end opposite the sensor and, depending on the rotational position of the teeth with respect to the light pipe, may be reflected by a tooth of the number sleeve back into the light pipe which guides the reflected light beam to the receiving portion of the sensor. The light pipe may be made of glass or polycarbonate. Reference is made to this document regarding disclosure of the working principle of the encoder system in conjunction with the drug delivery device.

Unpublished international patent applications PCT/EP2020/085728 and PCT/EP2020/085729 disclose similar modules for drug delivery devices having an encoder system comprising optical sensors with a chassis including a light pipe. In PCT/EP2020/085728 and PCT/EP2020/085729 the electronic module is configured for a detachable attachment to a drug delivery device and comprises respective fastening elements. The risk of an erroneous pairing of an electronic module with a drug delivery device is minimized by providing dedicated electronic modules to particular drug delivery devices.

Such drug delivery devices are typically manufactured in large scale such that an efficient and simple assembly is an important issue to keep production costs reasonably low.

It is an object of the present disclosure to provide improvements for electronic modules to be used with drug delivery devices allowing reasonable production and assembly efforts.

This object is solved for example by the subject matter defined in the independent claims. Advantageous embodiments and refinements are subject to the dependent claims. However, it should be noted that the disclosure is not restricted to the subject matter defined in the appended claims. Rather, the disclosure may comprise improvements in addition or as an alternative to the ones defined in the independent claims as will become apparent from the following description.

One aspect of the disclosure relates to an electronic module suitable for use with a drug delivery device. Such a drug delivery device may comprise a dose setting and drive mechanism which is configured to perform a dose setting operation for setting a dose to be delivered by the drug delivery device and a dose delivery operation for delivering the set dose. The electronic module preferably comprises at least one processor, e.g. a microcontroller, a sensor arrangement, a communication unit with a wireless communication interface, at least one electronic user feedback generator, a memory for storing measurement data, and a power source connected to the at least one microcontroller.

According to an aspect of the present disclosure, the electronic module is suitable for releasable attachment to the drug delivery device. The the module comprises a component which may be a chassis of the module. The electronic module may further comprise a cap, a printed circuit board assembly (PCBA) and a power source. For example, the component is a chassis rigidly attached to the cap and supporting the PCBA within the cap. This chassis component may have several different functions thereby reducing the number of component parts required for the module. This keeps production and assembly time and costs low. In the following, the component part will be mainly referred to as a chassis component part, however, the function of the component part being a chassis, e.g. for supporting the PCBA, is not mandatory. Rather, the present disclosure includes all types of component parts irrespective of the ability to have a chassis function.

Although all features and functions of the chassis component listed in the following may be implemented together in the chassis component, the present disclosure is not limited to such an embodiment. Rather, according to the present disclosure, the chassis component may comprise only one, preferably at least two of the following independent features and functions:
According to a first independent aspect of the present disclosure, the chassis component of the module may comprise at least one module lockout form adapted for mating abutment with a corresponding device lockout form of a dedicated drug delivery device. In other words, the electronic module may comprise the module lockout form as a mechanical coding feature to engage with the corresponding device lockout form as a mechanical counter coding feature provided at the proximal end of the dedicated drug delivery device. The term dedicated drug delivery device is used in the present disclosure to indicate that a drug delivery device mateches or mates with the respective module.

Generally, there may be provided a kit of numerous electronic modules, that distinguish by their respective module lockout form (mechanical coding). A first mechanical coding of a first electronic module distinguishes from a second mechanical coding of a second electronic module. For example, the first mechanical coding or first module lockout form comprises at least one of a first protrusion and a first recess. The second mechanical coding feature or second module locout form comprises at least one of a second protrusion and a second recess. The geometric shape of the first protrusion may distinguish from the geometric shape of the second protrusion. Additionally or alternatively, a position of the first protrusion in a plane transverse to the longitudinal direction distinguishes from a position of the second protrusion in the transverse plane. The same applies to the first and second recesses of first and second electronic modules. Further, the present disclosure also relates to a set of drug delivery devices that distinguish by their corresponding device lockout forms or mechanical (counter) codings. There may be provided at least a first drug delivery device with a first corresponding device lockout form or first mechanical counter coding and a second drug delivery device with a second corresponding device lockout form or second mechanical counter coding. The first module lockout form matches, i.e. mates, with the first corresponding device lockout form but does not match with the second corresponding device lockout form. Likewise, the second module lockout form only matches, i.e.mates, with the second corresponding device lockout form but does not match with the first corresponding device lockout form. The first and the second mechanical counter coding of first and second drug delivery devices distinguish by at least one of a geometric shape and a transverse position of the respective counter coding features, hence by their shape and/or transverse position of their protrusion or recess.

Mutual engagement of the mechanical coding and the counter mechanical coding is achieved when the respective lockout form mate, i.e. when the mechanical coding matches with the mechanical counter coding and when the electronic module is attached to the proximal end of the drug delivery device in a predefined fastening configuration.

In an example of the first aspect of the present disclosure the at least one module lockout form comprises a profiled protrusion or seat adapted for mating abutment with a corresponding contoured seat or protrusion of the device lockout form of said dedicated a drug delivery device. More specifically, in an example of the present disclosure, a drug delivery device comprises a button which is rotatable to select a dose and which is axially displaced for dispensing a dose. Such a button may be provided with a circular groove in a proximally facing end surface. This groove is adapted to receive at least one, e.g. two, protrusions of the module, e.g. specifically of the chassis component of the module. These protrusions constitute the module lockout forms. The groove is not rotationally symmetrical but comprises one or more inner blocking features as the corresponding device lockout form. The design of the module lockout form(s) and the corresponding device lockout form(s) is such that if a user attempts to fit a module to the incorrect device, it will simply come apart as soon as pressure is released.

In other words, the at least one module lockout form prevents attachment of the module on a non-mating drug delivery device.For example, in case that an electronic module comprising a module lockout form non-matching with the corresponding device lockout form of the drug delivery device, the lockout forms (coding features) might be located circumferentially offset. In the predefined fastening configuration, in which the module is fully attached to the drug delivery device, e.g. if fastening elements of the module engage with the correspondingly shaped counter fastening elements of the drug delivery device, the respective lockout forms may have no suitable counter form to run in. They may thus block and impede a proper arrangement and assembly of the electronic module on the drug delivery device.

As an alternative to such lockout forms on the chassis component, keying/blocking features may be provided on a separate component, e.g. on a casing or cap of the electronic module and/or on an optional adapter of the electronic module. Press fit, form fit, snug fit, force fit or other connection means may be used to connect the keying and/or blocking feature to the casing and/or to the chassis component.

According to a second independent aspect of the present disclosure, the chassis component of the module may comprise at least one attachment element for releasable attachment of the module on a drug delivery device. For example, the at least one attachment element for releasable attachment of the module on a drug delivery device comprises at least one elastically deformable arm with a snap protrusion or recess for releasable engagement with a corresponding snap recess or protrusion of the drug delivery device. Generally, the electronic module may be used with several drug delivery devices of equal or of the same type. The assembly or fastening of the electronic module to the drug delivery device is hence only a temporary assembly. A mutual fastening of the electronic module and the drug delivery device may require a distally directed movement of the electronic module from a pre-assembly configuration into a final assembly configuration, the latter of which may be a predefined fastening configuration.

In other words, the electronic module may comprise a fastening or attachment element configured to mechanically engage with a complementary-shaped counter fastening or attachment element of the drug delivery device in the predefined fastening configuration. The fastening or attachment element and the counter fastening or attachment element may define the predefined fastening configuration, in which the electronic module is attachable, coupleable or connectable to the drug delivery device. A position and/or a geometric shape of the fastening or attachment element of the electronic module typically matches with the geometric shape and/or with the position of the counter fastening or attachment element of the drug delivery device. A mutual assembly, hence arranging the electronic module on the drug delivery device in the predefined fastening configuration requires that the fastening or attachment element mechanically engages with the complementary-shaped counter fastening or attachment element. When the fastening or attachment element and the complementary-shaped counter fastening or attachment element are in mechanical engagement, the electronic module is in a predefined orientation as well as in a predefined position relative to the drug delivery device.

The fastening or attachment element may comprise a clip feature and may form a clip connection with the correspondingly or complementary-shaped counter fastening or attachment element. Hence, the counter fastening or attachment element of the drug delivery device may also comprise a clip feature and may contribute to a click connection between the electronic module and the drug delivery device. With other examples, the fastening or attachment element may be configured to establish a friction fit or a force fit with the complementary-shaped counter fastening or attachment element of the drug delivery device.

More specifically, the chassis component may comprise two flexible clips as attachment elements protruding distally from an internal rim of the chassis component, which clips axially retain and rotationally orientate the module on the drug delivery device. The drug delivery device may comprise a button having two apertures into which the two flexible clips snap. For example, the chassis component may have at least one, e.g. two, elastically deflectable snap hook(s) suitable for engagement with corresponding snap recess(es) in a button of the drug delivery device.

As an alternative to such attachment element(s) on the chassis component, fastening features may be provided on a separate component, e.g. on a casing or cap of the electronic module and/or on an optional adapter of the electronic module.

According to a third independent aspect of the present disclosure, the chassis component of the module may comprise at least one light pipe for guiding a light beam from a light source to a reflective surface of the drug delivery device and from said reflective surface to a light detector sensor. For example, the at least one light pipe may be a protrusion in the shape of a conical frustum. As an alternative, the at least one light pipe may be a protrusion in the form of a truncated pyramid. Still further alternatives may include a cylindrical shape or an elongate cuboid shape.

The at least one light pipe may have two opposite end faces and at least one sidewall, wherein at least one of the two opposite end faces have a surface roughness higher than the surface roughness of the at least one sidewall. This facilitates entry or exit of light through the end faces, while light is reflected by the at least one sidewall. In other words, the at least one light pipe may comprise a border surface that may guide an electromagnetic radiation by total reflection.

In an exemplary embodiment, one end face of the light pipe may have the same surface finish as the sides of the light pipe, e.g. polished, so only one of the end faces has a higher roughness than the sidewalls. Other combinations of surface finish could be used successfully.

In an example of the present disclosure, the module may comprise at least two light pipes protruding axially in the same direction from an inner portion of the chassis component. More specifically, the light pipes may be arranged on a circular rim or a portion thereof such that the light pipes may be inserted into apertures of a circular groove in a button of the drug delivery device. The light pipes may be parallel to each other and/or the respective central axes of the light pipes may be parallel to each other.

The light pipe(s) may extend axially over the module lockout form(s) and/or over the attachment element(s). In other words, the length of the light pipe(s) in the distal direction may be such that the light pipe(s) may enter into a button of the drug delivery device, when the module is fully attached to the drug delivery device, i.e. with the lockout forms in engagement and/or the attachment elements fully connected.

As an alternative to the provision of light pipe(s), an optical sensor may be located in the module such that an encoder, e.g. located in the vicinity of the interface between the module and the drug delivery device, may be detected by the sensor.

According to a fourth independent aspect of the present disclosure, the chassis component of the module may comprise at least one light guide for guiding a light beam from a light source to a user feedback surface of said component adapted to emit light. The user feedback surface may be a light-emitting region, i.e. a surface visible from the outside of the module, such that light signals may be used to indicate e.g. a status of the module. The user feedback surface may have an annular shape, e.g. in the form of an outwardly facing light ring of the chassis component.

For example, the at least one light guide may comprise an annular skirt having at least one entry surface and the user feedback surface which is radially facing outwardly from the annular skirt. The at least one entry surface and the user feedback surface may have a surface roughness higher than the surface roughness of the annular skirt. This facilitates entry or exit of light through the entry surface and the user feedback surface, while light is reflected by the annular skirt. In other words, the at least one light guide may comprise the annular skirt as a border surface that may guide an electromagnetic radiation by total reflection.

The surface roughness of the light pipe(s) and/or the light guide(s) may be chosen such that an area intended for light entry and/or light exit, e.g. the two opposite end faces of a light pipe or the entry surface and the user feedback surface of a light guide, have a textured finish as surface finish, particularly a textured finish according to the D3, D2 or D1 standard of the Society of Plastics Industry SPI. The textured finish may be a finish having a slight roughness or diffusivity such SPI-D3 or even finer, for example characterized as having features sizes of about 1 µm, particularly nearly equal to the central wavelength of an infrared (IR) - light emitting diode (LED) sensor package, which may be applied as optical sensor. In a light pipe, the encoder side surface may have a mirrored finish; the encoder side surface may comprise an antireflection coating. A mirrored finish and an antireflection coating may reflect interfering light and, thus, may prevent that the interfering light enters the light pipe and may reduce the signal-to-noise ratio.

Further, the surface roughness of the light pipe(s) and/or the light guide(s) may be chosen such that an area intended for guiding light, e.g. a sidewall of a light pipe or the skirt of a light guide, have a mirror finish. This finish may promote total internal reflection (TIR) and efficacy of the light pipe. The mirror finish surface may be used in combination with the textured finish of the area intended for light entry and/or light exit whereby the roughness of the area intended for light entry and/or light exit reduces the amount of TIR, e.g. at the encoder-side surface, as compared to the mirror finish. In further examples of the optical guiding means, e.g. light pipe(s) and/or the light guide(s), an area intended for guiding light may comprise one or more coatings, wherein the outermost coating may be non-transparent for the guided radiation, or wherein all coatings may be transparent for the guided radiation and an optical refractive index of each of the transparent coatings is smaller than the optical refractive index of the chassis component itself. This may further improve the light guiding, e.g. by the at least one light pipe, and may also reduce influences of interfering light for example from outside of the light pipe. Thus, the signal-to-noise-ratio may be improved.

In the electronic module the annular skirt may comprises at least two, e.g. four, entry surfaces which are each formed in a respective recess for receiving a light source, e.g. a respective LED. In an example of the present disclosure, the light guide may be used to indicate different operational states of the module. Such states may include a state in which the module is attempting to synchronise with another external device via a wireless communication interface of the module, e.g. by a flash of all LEDs on a light ring, and a state in which the module is attempting to pair with another external device via a wireless communication interface of the module.

As an alternative to the provision of a light guide in the chassis component, the module may indicate a state by means of one or more light source(s) directly visible from the outside or visible through a window. Still further feedback alternatives may include a sound generator and/ or a vibration motor. Such other user signals may be provided by the module, e.g. indicating operation of the sensor, start and/or end of dose dialling and/or dose dispensing, expiry of a dwell time or the like. However, it is not required that the module itself generates a user feedback. As an alternative or in addition, such a feedback may further be generated by the drug delivery device.

According to a fifth independent aspect of the present disclosure, the chassis component of the module may comprise at least one elastically deformable switch arm. For example, the at least one elastically deformable switch arm may extend in a circumferential direction. Alternatively or additionally, the at least one elastically deformable switch arm may comprise a free end and may be deflectable with respect to the chassis component in order to actuate an electronic switch.

The present disclosure is further directed at a method of waking an electronic encoding module that is configured as a re-usable clip-on module for a drug delivery device, e.g. an injection device. This method includes the step of waking the electronic module on or shortly before the beginning of dose delivery. This limits power consumption by having the capture system active for the shortest possible time. This is particularly useful for systems such as an optical encoder, where the power consumption of the IR-LED sensors accounts for a significant proportion of the total power consumption of the electronic module. The method may further comprise the step of waking the module by actuation of a switch by means of a switch arm which is deflected as a button of the injection device is moved axially with respect to a stationary component part, e.g. the housing, of the device when starting dose dispensing. There are a number of embodiments of such a wake switch.

According to a further independent aspect of the present disclosure, a switch, e.g. alow-force micro-switch, may be mounted onto the underside of the electronic module, e.g. on the distally facing side of the PCBA. This micro-switch may have over-travel beyond its switching point. For example, a switch such as a Panasonic ESE16J001 may be suitable. The switch may be actuated by a flexible switch arm formed in the chassis component of the module. This flexible switch arm may be configured in such a way as to fit within an annular groove in the rear of the button component of the injection device which button may be intended to form the interface to the electronic module. A feature on the flexible switch arm may be designed to pass through an aperture in the button and contact against a component which does not move axially during clutch disengagement of the mechanism at the beginning of dose dispense. For example, the feature on the flexible switch arm may be designed to bear against a drive sleeve component of the injection pen device.

In other words, when the top face of the electronic module is pushed axially to initiate dose dispense, the button of the injection pen may move distally to disengage the clutch features. The resulting relative motion between the electronic module and the drive sleeve may cause the flexible switch arm of the chassis component to deflect and bear against the micro-switch mounted on the underside of the PCBA. For example, the micro-switch may be configured to switch and wake the electronic module after a short travel (before the clutch is fully disengaged) but must also tolerate sufficient over-travel to allow the full stroke of the dose button to disengage the clutch, after the electronic switch has been made.

In order to protect against water and dirt ingress, the module may be configured with an elastomeric seal component, which is mounted between the PCBA and the flexible switch arm. The elastomeric component forms a compressive face seal between the PCBA and chassis component, preventing water and dirt ingress. This elastomeric seal component is located between the flexible switched arm (formed on the chassis component) and the micro-switch (mounted on the PCBA). Due to the flexible nature of the sealing component, it is able to deflect and transmit axial load from the switch arm to the micro-switch-permitting normal operation of the wake switch as described above.

As an alternative to the provision of the elastically deformable switch arm, a switch may be located at the proximal end of the PCBA, i.e. facing away from the drug delivery device, and this switch may be actuated by an elastically deformable portion in the cap as a user presses the cap at the beginning of dose dispensing. Further alternatives include waking of the module using the optical sensor or by means of a separate switch arm mounted on the drug delivery device and/or on another component part of the module.

According to a sixth independent aspect of the present disclosure, the chassis component of the module may be a unitary component part injection molded from a thermoplastic polymer material which is highly transparent to light, e.g. to visible light and/or to IR light, and which can undergo elastic deformations. For example, the chassis component may be made from a polycarbonate material, such as Covestro Makrolon 2458, by an injection moulding process. [

According to a seventh independent aspect of the present disclosure, the chassis component with the exception of the user feedback surface, may be encased by the cap wherein the printed circuit board assembly (PCBA) and the power source are interposed between the cap and the component.

According to an eighth independent aspect of the present disclosure, the chassis component may have a substantially cylindrical outer shape with the radially facing user feedback surface forming a distal end wherein the component may comprise an inner rim with the at least one module lockout form, the at least one attachment element and the at least one light pipe extending distally from the rim and at least one collar portion extending proximally from the rim.

The sensor arrangement may be connected to the at least one processor and operable to generate measurement data indicative of the dose setting operation and/or the dose delivery operation. The sensor arrangement may comprise one or more electrical switch(es) and/or may include opical and/or capacitive and/or acoustic sensors for detecting a movement of one or more component parts of the dose setting and drive mechanism of the drug delivery device. In one example, the sensor arrangement comprises at least one light source, e.g. an LED, and at least one light sensor, e.g. a photo detector. The sensor arrangement may be part of an encoding or motion sensing unit designed and working as described in unpublished EP 20315066.9 and EP 20315357.2, the disclosure of which is incorporated herein by reference.

The communication unit with the wireless communication interface may be connected to the at least one processor and operable to establish communication with another device and to transfer data to another device. Despite the fact that establishing wireless communication typically involves a transfer of data, with respect to the present disclosure, establishing communication, which may include for example the process of broadcasting advertising packets, scanning for such advertising packets and pairing two devices, is to be distinguished from the data transfer itself which is defined to occur only after successful pairing and typically involves significantly higher data transfer volume compared to establishing wireless communication like a manual syncronisation and/or a pairing.

The communication unit for communicating with another device may comprise a wireless communications interface for communicating with another device via a wireless network such as Wi-Fi or Bluetooth^{®}. In addition, the communication unitor may comprise an interface for a wired communications link, such as a socket for receiving a Universal Series Bus (USB), mini-USB or micro-USB connector. Preferably, the electronic system comprises an RF, WiFi and/or Bluetooth^{®} unit as the communication unit. The communication unit may be provided as a communication interface between the module or the drug delivery device and the exterior, such as other electronic devices, e.g. mobile phones, personal computers, laptops and so on. For example, measurement data, i.e. dose data, may be transmitted by the communication unit to the external device. The dose data may be used for a dose log or dose history established in the external device. In the following, the wireless communications interface will be described referring to the example of Bluetooth^{®} communication between the module and a smartphone. However, this is not to be understood as a limitation excluding the above-mentioned alternatives of wireless communication.

The memory for storing measurement data, e.g. dose data, may be a separate memory or may be part of a main memory of the electronic module. These are controlled by the processor, which may for instance be the at least one microcontroller, a Digital Signal Processor (DSP), Application Specific Integrated Circuit (ASIC), Field Programmable Gate Array (FPGA) or the like. According to an aspect of the disclosure, the processor executes program code (e.g. software or firmware) stored in a program memory, and uses a main memory, for instance to store intermediate results, like dose data. Main memory may also be used to store a logbook on performed ejections/injections based on measurement data. Program memory may for instance be a Read-Only Memory (ROM), and main memory may for instance be a Random Access Memory (RAM).

The power source is connected to the processor and powers the processor and other components, like the sensor arrangement, the communication unit and the at least one electronic user feedback generator by way of a power supply. The power source may be a non-rechargeable, non-user replaceable coin cell.

A potting compound or filling layer may be applied on the chassis and/or on the PCBA preventing ingress of dust and water to the conductive areas of the PCBA.

The coin cell may be secured and connected to the PCBA by means of a power source clip which may be attached to the chassis component. The clip may have a curved form in its unbiased (unstressed) state and may be deformed when mounted onto the chassis component. The chassis component may have corresponding snap features for attachment of the clip, in particular of the free ends of the clip. The clip may consist of an elastically deformable and electrically conductive material, e.g. a metal.

An additional or alternative switch may be provided on the PCBA. This switch may be actuated if the module is fully and correctly fitted onto the button, e.g. by contact between the distal switch surface and a proximally facing button surface. Such a switch may be used to activate the processor or components thereof, e.g. from a no-power or sleeping mode of the module when the module is not attached to the device.

The present invention further pertains to a drug delivery device comprising the electronic module as described above. According to a further independent aspect of the present disclosure, the drug delivery device may comprise a button located at a proximal end thereof. The button may be rotatable by a user to dial (select) a dose. In addition, the button may be axially displaceable, e.g. in the distal direction, to perform a dispensing stroke. In an example, the button is adapted to attach the electronic module to the drug delivery device. More specifically, the button may comprise attachment features, e.g. one or more snap recess(es) for receiving and engaging attachment features of the module. Further, the button may comprise device lockout form(s) adapted to mate with lockout forms of the module. This may prevent attachment of a nun suitable module to the device. Still further, the button may be provided with one or more aperture(s) permitting one or more light pipe(s) and/or a portion of a switch arm to enter into the drug delivery device.

The drug delivery device for delivery of a medicament may comprise a dose setting and drive mechanism which is configured to perform a dose setting operation for setting a dose to be delivered by the drug delivery device and a dose delivery operation for delivering the set dose and which comprises a first member. This dose setting and drive mechanism may comprise the button. The drug delivery device may further comprise a container receptacle which is releasably attached to the dose setting and drive mechanism. As an alternative, the container receptacle may be permanently attached to the dose setting and drive mechanism. The container receptacle is adapted to receive a container, e.g. a cartridge, containing a medicament.

Further additional and/or alternative examples of the present invention are defined in the following:
In one example of the electronic module (11) for releasable attachment to a drug delivery device (1), the module (11) comprises a component (320) which comprises at least two of the following features:
- at least one module lockout form (323) adapted for mating abutment with a corresponding device lockout form (21) of a dedicated drug delivery device (1),
- at least one attachment element (324) for releasable attachment of the module (11) on a drug delivery device (1),
- at least one light pipe (325) for guiding a light beam from a light source (121) to a reflective surface (24) of the drug delivery device (1) and from said reflective surface (24) to a light detector sensor (122),
- at least one light guide (321) for guiding a light beam from a light source (141) to a user feedback surface (322) of said component (320) adapted to emit light,
- at least one elastically deformable switch arm (326, 327),
- the component (320) is a unitary component part injection molded from a polycarbonate material.

In a further example, the electronic module further comprises a cap (310), a printed circuit board assembly (330) and a power source (160), wherein the component (320) is a chassis rigidly attached to the cap (310) and supporting the printed circuit board assembly (330) within the cap (310).

In a further example, the at least one module lockout form (323) of the electronic module comprises a profiled protrusion or seat adapted for mating abutment with a corresponding contoured seat or protrusion of the device lockout form (21) of said dedicated drug delivery device (1).

In a further example of the electronic module, the at least one module lockout form (323) prevents attachment of the module (11) on a non-mating drug delivery device (1).

In a further example of the electronic module, the at least one attachment element (324) for releasable attachment of the module (11) on a drug delivery device (1) comprises at least one elastically deformable arm with a snap protrusion or snap recess for releasable engagement with a corresponding snap recess (22) or snap protrusion of the drug delivery device (1).

In a further example of the electronic module, the at least one light pipe (325) is an elongate cuboid shaped protrusion or a protrusion in the shape of a conical frustum having two opposite end faces and at least one sidewall, wherein at least one of the two opposite end faces have a surface roughness higher than the surface roughness of the at least one sidewall.

In a further example, the electronic module further comprises at least two light pipes (325) protruding axially in the same direction from an inner portion (328) of the component (320).

In a further example of the electronic module, the at least one light guide comprises an annular skirt (321) having at least one entry surface (328) and the user feedback surface (322) which is radially facing outwardly from the annular skirt (321), wherein the at least one entry surface (328) and the user feedback surface (322) have a surface roughness higher than the surface roughness of the annular skirt (321).

In a further example of the electronic module, the annular skirt (321) comprises at least two, e.g. four, entry surfaces which are each formed in a respective recess for receiving a light source (141).

In a further example of the electronic module, the at least one elastically deformable switch arm (326) extends in a circumferential direction.

In a further example of the electronic module, the at least one elastically deformable switch arm (326) comprises a free end (327) and is deflectable with respect to the component (320) in order to actuate an electronic switch (331).

In a further example of the electronic module, the component (320), with the exception of the user feedback surface (322), is encased by the cap (310) and wherein the printed circuit board assembly (330) and the power source (160) are interposed between the cap (310) and the component (320).

In a further example of the electronic module, the component (320) has a substantially cylindrical outer shape with the radially facing user feedback surface (322) forming a distal end and wherein the component (320) comprises an inner rim (328) with the at least one module lockout form (323), the at least one attachment element (324) and the at least one light pipe (325) extending distally from the rim (328) and at least one collar portion (329) extending proximally from the rim.

In a further example, the electronic module further comprises:
- at least one processor (110),
- a sensor arrangement (120) connected to the at least one processor (110) and operable to generate measurement data indicative of a dose setting operation and/or a dose delivery operation of the dedicated drug delivery device (1),
- a communication unit (130) with a wireless communication interface connected to the at least one processor (110) and operable to establish communication with another device (200) and to transfer data to said other device (200),
- at least one electronic user feedback generator (140) connected to the at least one processor (110) and operable to generate a feedback signal, and
- a memory (150) for storing measurement data.

In a further example, a drug delivery device (1) for delivery of a medicament comprises a dose setting and drive mechanism (23), which is configured to perform a dose setting operation for setting a dose to be delivered by the drug delivery device (1) and a dose delivery operation for delivering the set dose and which comprises a first member (20), and a container receptacle (14) which is permanently or releasably connected to the dose setting and drive mechanism and which is adapted to receive a container containing a medicament. In this example, the drug delivery device comprises the electronic module (11) according to any one of the aforementioned examples.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The terms "axial", "radial", or "circumferential" as used herein may be used with respect to a main longitudinal axis of the device, the cartridge, the housing or the cartridge holder, e.g. the axis which extends through the proximal and distal ends of the cartridge, the cartridge holder or the drug delivery device.

Non-limiting, exemplary embodiments of the invention will now be described with reference to the accompanying drawings, in which:
- Figure 1: shows an embodiment of a drug delivery device;
- Figure 2: schematically illustrates an embodiment of an electronic module for a drug delivery device;
- Figure 3: schematically illustrates a sectional view of an embodiment of an electronic module for a drug delivery device;
- Figure 4: schematically illustrates a further sectional view of the electronic module of Figure 3 attached to a drug delivery device;
- Figure 5a: schematically illustrates a perspective view of the electronic module of Figure 3;
- Figure 5b: schematically illustrates a perspective view of a further electronic module;
- Figures 6a-c: schematically illustrate views of embodiments of a button of a drug delivery device for attachment of the electronic module of Figure 3;
- Figures 7a, b: schematically illustrate perspective views of a chassis component of the electronic module of Figure 5b;
- Figure 8: is a table depicting different lockout forms;
- Figure 9: schematically illustrates a perspective view of another detail of an electronic module;
- Figures 10a-10d: schematically illustrate a clip in an unstressed condition, in a deflected (mounted) condition, prior to attchmanet to the chassis and after attachment to the chassis ; and
- Figure 11: schematically illustrates a sectional view of a further electronic module attached to a drug delivery device.

In the Figures, identical elements, identically acting elements or elements of the same kind may be provided with the same reference numerals.

In the following, some embodiments will be described with reference to an insulin injection device. The present disclosure is however not limited to such application and may equally well be deployed with injection devices that are configured to eject other medicaments or drug delivery devices in general, preferably pen-type devices and/or injection devices.

Embodiments are provided in relation to injection devices, in particular to variable dose injection devices, which record and/or track measurement data on doses delivered thereby. These data may include the size of the selected dose and/or the size of the actually delivered dose, the time and date of administration, the duration of the administration and the like. Features described herein include power management techniques (e.g. to facilitate small batteries and/or to enable efficient power usage).

Certain embodiments in this document are illustrated with respect to the injection device disclosed in EP 2 890 435 where an injection button and grip (dose setting member or dose setter) are combined. The injection button may provide the user interface member for initiating and/or performing a dose delivery operation of the drug delivery device. The grip or knob may provide the user interface member for initiating and/or performing a dose setting operation. These devices are of the dial extension type, i.e. their length increases during dose setting. Other injection devices with the same kinematical behaviour of the dial extension and button during dose setting and dose expelling operational mode are known as, for example, the Kwikpen^{®} device marketed by Eli Lilly and the Novopen^{®} 4 device marketed by Novo Nordisk. An application of the general principles to these devices therefore appears straightforward and further explanations will be omitted. However, the general principles of the present disclosure are not limited to that kinematical behaviour. Certain other embodiments may be conceived for application to Sanofi's SoloSTAR^{®} injection device where there are separate injection button and grip components / dose setting members. Thus, there may be two separate user interface members, one for the dose setting operation and one for the dose delivery operation.

"Distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

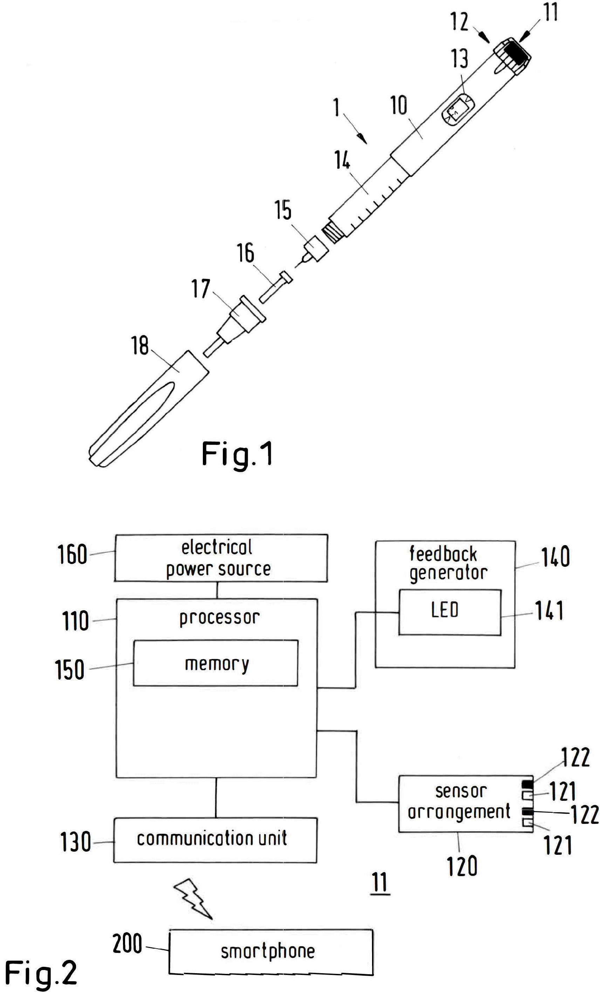

Figure 1 is an exploded view of a medicament delivery device or drug delivery device. In this example, the medicament delivery device is an injection device 1, e.g. a pen-type injector, such an injection pen disclosed in EP 2 890 435.

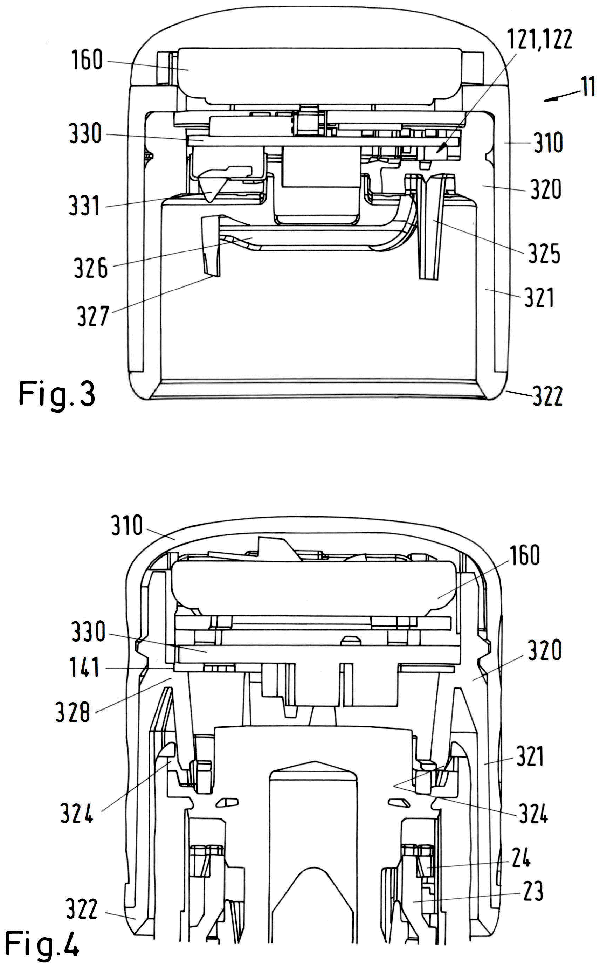

The injection device 1 of Figure 1 is an injection pen that comprises a housing 10 and contains a container 14, e.g. an insulin container, or a receptacle for such a container. The container may contain a drug. A needle 15 can be affixed to the container or the receptacle. The container may be a cartridge and the receptacle may be a cartridge holder. The needle is protected by an inner needle cap 16 and either an outer needle cap 17 or another cap 18. An insulin dose to be ejected from injection device 1 can be set, programmed, or 'dialled in' by turning a button or dial grip (dosage knob) 12, and a currently programmed or set dose is then displayed via dosage window 13, for instance in multiples of units. The indicia displayed in the window may be provided on a number sleeve 23 or dial sleeve (partially depicted in Figure 4 with a ring of teeth 24). For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a dial sleeve assembly that is configured to move when the button or dial grip 12 is turned, to provide a visual indication of a currently set dose. The button or dial grip 12 is rotated on a helical path with respect to the housing 10 when setting a dose.

In this example, the button or dial grip 12 includes one or more formations to facilitate attachment of a data collection device. Especially, the button or dial grip 12 may be arranged to attach or integrate an electronic (button) module 11 onto the button or dial grip 12. As an alternative, the dial grip may comprise such a button module of an electronic system.

The injection device 1 may be configured so that turning the button or dial grip 12 causes a mechanical click sound to provide acoustic feedback to a user. In this embodiment, the button or dial grip 12 also acts as an injection button. When needle 15 is stuck into a skin portion of a patient, and then button or dial grip 12 and/or the attached module 11 is pushed in an axial direction, the insulin dose displayed in display window 13 will be ejected from injection device 1. When the needle 15 of injection device 1 remains for a certain time in the skin portion after the button or dial grip 12 is pushed, the dose is injected into the patient's body. Ejection of the insulin dose may also cause a mechanical click sound, which may be different from the sounds produced when rotating the button or dial grip 12 during dialing of the dose.

In this embodiment, during delivery of the insulin dose, the button or dial grip 12 is returned to its initial position in an axial movement, without rotation, while the dial sleeve assembly is rotated to return to its initial position, e.g. to display a dose of zero units. Figure 1 shows the injection device 1 in this 0U dialled condition. As noted already, the disclosure is not restricted to insulin but should encompass all drugs in the drug container 14, especially liquid drugs or drug formulations.

Injection device 1 may be used for several injection processes until either the insulin container 14 is empty or the expiration date of the medicament in the injection device 1 (e.g. 28 days after the first use) is reached. In the case of a resuable device, it is possible to replace the insulin container.

Furthermore, before using injection device 1 for the first time, it may be necessary to perform a so-called "prime shot" to remove air from insulin container 14 and needle 15, for instance by selecting two units of insulin and pressing button or dial grip 12 while holding injection device 1 with the needle 15 upwards. For simplicity of presentation, in the following, it will be assumed that the ejected amounts substantially correspond to the injected doses, so that, for instance the amount of medicament ejected from the injection device 1 is equal to the dose received by the user. Nevertheless, differences (e.g. losses) between the ejected amounts and the injected doses may need to be taken into account.

As explained above, the button or dial grip 12 also functions as an injection button so that the same component is used for dialling/setting the dose and dispensing/delivering the dose. As an alternative (not shown), a separate injection button may be used which is axially displaceable, at least a limited distance, relative to a dial grip 12 to effect or trigger dose dispensing.

In the following, an electronic module 11 according to the present disclosure will be described with respect to exemplary embodiments and with reference to Figures 1 to 6. In Figure 1, the electronic module 11 is depicted as being integrated in the proximal end of the injection device 1, specifically integrated into the dial grip/dose button 12. As an alternative, the electronic module 11 may be a separate component part which may be permanently or releasably attached to the injection device 1, e.g. to the grip/dose button 12.

As depicted in Figure 2, an exemplary electronic module comprises a processor 110, a sensor arrangement 120, a communication unit 130, an electronic user feedback generator 140, a memory 150, and a power source 160.

In the example depicted in Figure 2, the sensor arrangement 120 is connected to the processor 110 and operable to generate measurement data indicative of the dose setting operation and/or the dose delivery operation. For this purpose the sensor arrangement comprises a LED 121 and a photo detector 122 together forming an optical sensor. Alternative sensor types could be implemented in addition to LED 121 and photo detector 122 or as an alternative thereto. Such alternative sensor types may include but are not limited to optical sensors, acoustic sensors, capacitive sensors, electrical switches.

The communication unit 130 comprises with a wireless Bluetooth^{®} communication interface connected to the processor 110 and operable to establish communication with another (external) device, e.g. a smartphone 200. Further, the communication unit 130 is operable to transfer data, e.g. measurement data, to said other device 200.

The electronic user feedback generator 140 connected to the processor 110 and operable to generate a feedback signal to a user. In the exemplary arrangement of Figure 2, the electronic user feedback generator 140 comprises a LED 141 for generating optical feedback signals. In addition to the LED 141 or as an alternative to the LED 141, the electronic user feedback generator 140 may comprise a sounder and/or a vibration motor.

The memory 150 is adapted for storing measurement data and is connected to the processor 110 or is integrated into the processor 110. The power source 160 is connected to the processor 110. For example, the power surce 160 is a non-rechargeable, non-user replaceable coin cell.

Turning now to Figures 3 to 7b, the electronic module 11 comprises a cap 310, an inner component part 320, e.g. a chassis component, a printed circuit board assembly (PCBA) 330 and the power source 160 in the form of a coin cell.

The cap 310 may be a cup-shaped component with a cosed proximal end (upper end in Figures 3 and 4), a closed skirt which may have a serration or the like surface structure, and an open distel end facing towards the drug delivery device 1. The cap forms an outer shell for the module 11 and houses the PCBA 330, the coin cell 160 and at least a portion of component 320.

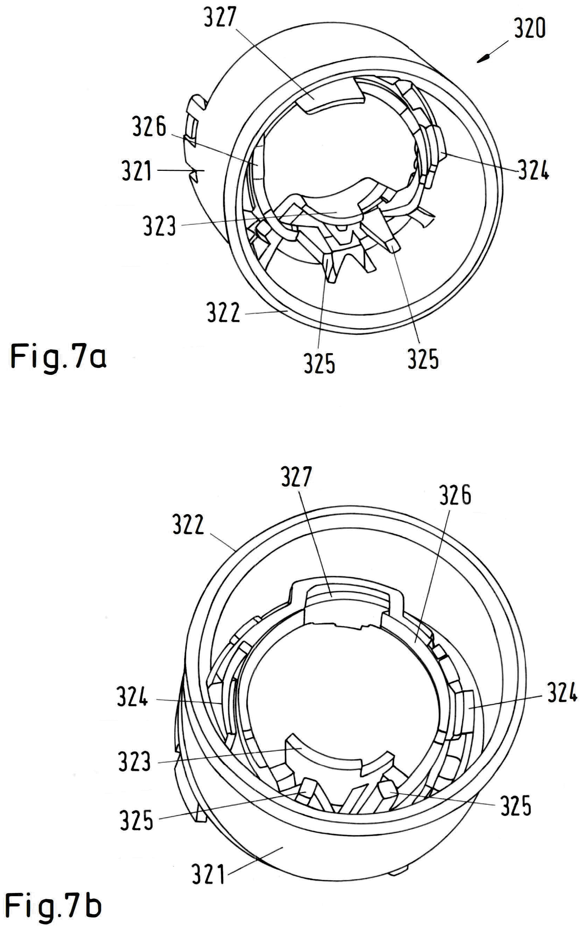

The component 320 which is depicted in Figures 7a and 7b from different sides is made from a transparent material, e.g. injection moulded from a polycarbonate material. The component 320 has an outer skirt 321 which is substantially cylindrical and fits into the space defined by cap 310. The skirt 321 may be provided with a circumferential outer bead and/or a circumferential outer groove for rigid attachment with a corresponding bead and/or groove structure of the cap 310. The annular distal end face of the skirt 321 forms a user feedback surface 322 suitable to emit light which enters the component 320 e.g. from an LED 141 provided e.g. on the PCBA 330. Thus, the skirt 321 acts as a light guide. As mentioned above, the surface roughness of the skirt 321 and the user feedback surface 322 may be adapted to enhance or permit the light guiding function. The user feedback surface 322 extends axially beyond the distal end of the cap 310 and may have an outer diameter similar to that of the cap 310. Thus, the user feedback surface 322 is visible from the outside of the module 11. The inner surface of the skirt 321 may be provided with information relating to the module 11 itself and/or to the device 1 to be used with the module 11.

Figures 3 and 4 show that the component 320 is provided with a rim 328 facing inwardly from the skirt 321. This rim 328 supports the PCBA 330 and the coin cell 160. For this purpose, at least one collar portion 329 may extend proxmally from the rim 328. Further, this rim 328 may comprise one or more recess(es) for receiving an LED 141 mounted on the PCBA 330.

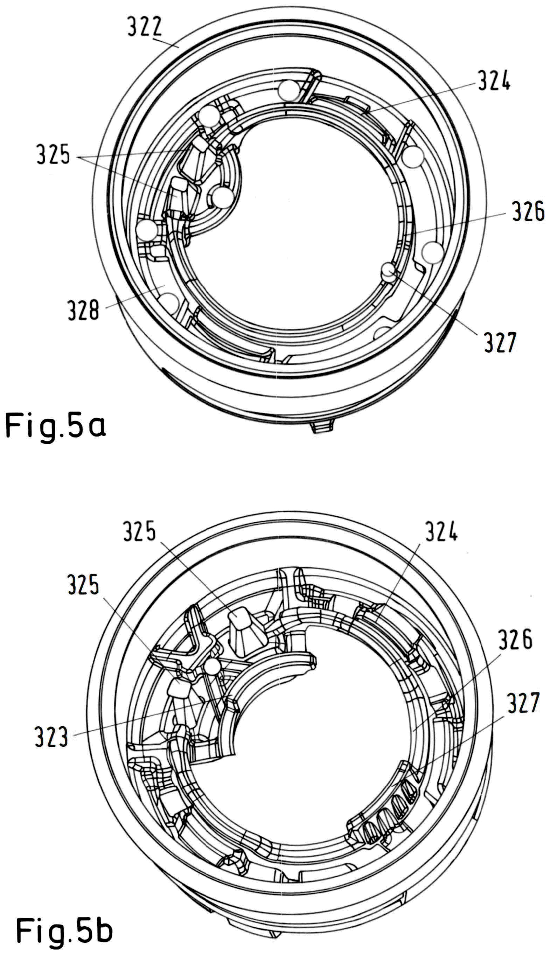

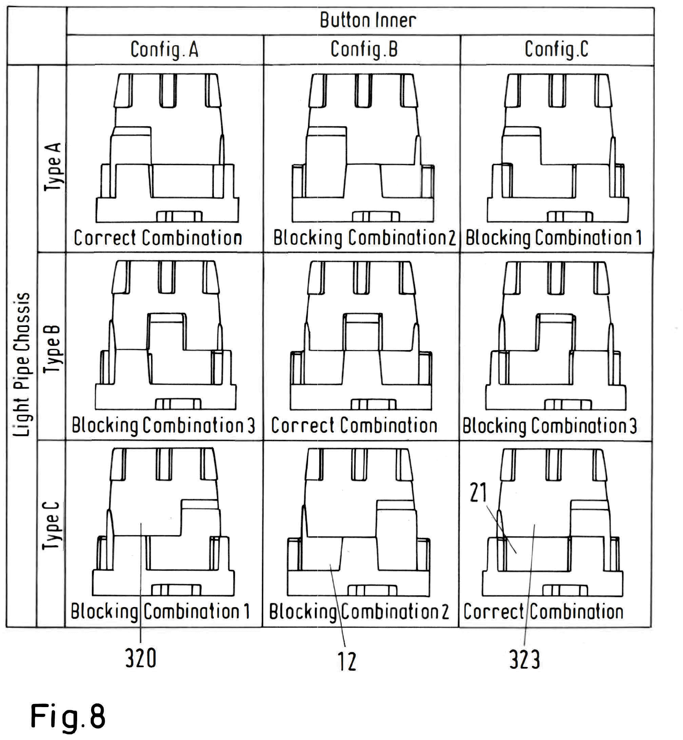

Figures 5a, 5b, 7a and 7b show a module lockout form 323 of the component 320 which comprises a profiled protrusion adapted for mating abutment with a corresponding contoured seat of a device lockout form 21 of a dedicated a drug delivery device 1, specifically of a button 12 of a drug delivery device 1. The function of the module lockout form 323 becomes apparent from the table of Figure 8 which depicts three different types of module lockout forms 323 in combination with three different types of corresponding device lockout forms 21 of button 12. The respective lockout frms 323 and 21 are designed such that only one specific module lockout form 323 mates with a specific corresponding device lockout form 21, thereby permitting attachment of the module 11 on the button 12 of the respective dedicated drug delivery device 1. However, if a user attempts to attach a module 11 to an incorrect drug delivery device 1, full attachment is prevented by non-mating module lockout form 323 and corresponding device lockout form 21. While Figure 8 shows three types of modules 11 and three types of drug delivery devices 1, different numbers of mating pairs of a module 11 and a dedicated drug delivery device 1 may be chosen.

The module 11 may be releasably fixed on the drug delivery device 1 by means of attachment elements 324 formed on component 320. Figures 5a, 5b, 7a and 7b show a pair of these attachment elements 324 in the form of elastically deformable snap hooks which may engage with corresponding recesses 22 formed in a disally facing groove 25 of the button/dial grip 12. The attachment elements 324 extend distally from the rim 328 of the component 320 The module 11 is attached rigidly to the button 12, meaning that it moves rotationally and axially with the button 12 at all times. Thus, during dialling, as the button 12 moves outwards on a helical path, along with the drive sleeve and the number sleeve 23, during this phase the module moves helically with the button 12, drive sleeve and number sleeve 23 on the same path.

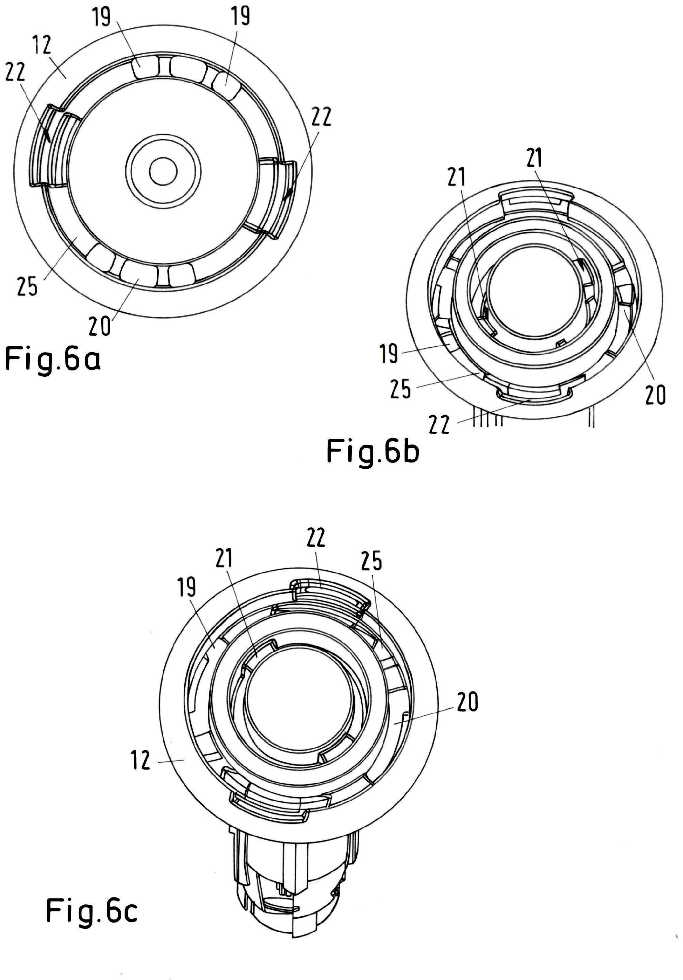

The component 320 further comprises two light pipes 325 which are elongate cuboid shape protrusions extending from rim 328 distally. The light pipes 325 have two opposite end faces adapted to permit light entry and light exit. The sidewalls of the light pipes 325 form a border surface that guides an electromagnetic radiation by total reflection. When the modue 11 is attached to the button 12 of the drug delivery device 1, the light pipes 325 extend through apertures 19 (see Figures 6a, 6b) in the groove 25 in the distal end face of button 12 as shown in Figure 4.

The sensor arrangement 120 with LED 121 and photo detector 122 is located at or near the proximal end face of each light pipe 325 on the PCBA 330. Thus, a light beam emitted from LED 121 may enter the light pipe 325, is guided distally, exits the light pipe 325 at its distal end, is reflected by a tooth 24 of number sleeve 23 (depending on the rotational position of the number sleeve 23) enters back into light pipe 325 and exits the light pipe at its proximal end to be detected by photo detector 122. On the other hand, if the number sleeve 23 is in a rotational position such that none of the reflective teeth 24 is located beneath the distal end of a respective light pipe 325, a light beam exiting the light pipe 325 is not reflected and, hence, is not detected by photo detector 122. Time shifted emission of light signals from the LEDs 121 may be used to detect rotation of the number sleeve 23 which is indicative of an amount of the dose dispensed from the drug delivery device 1. Thus, teeth 24 of number sleeve 23 act as an encoder reflecting or not reflecting light depending on the relative rotational position of the teeth.

Still further, component 320 comprises an elastically deformable swich arm 326 having an elongated distally extending free end 327. Two different designs of the free end 327 are depicted in Figures 5a and 5b. The switch arm 326 extends substantially circumferentially on a diameter on which the light pipes 325 are arranged. As depicted in Figures 5a and 5b, the switch arm 326 may have the form of an open ring hinged with its two ends to the rim 328. The free end 327 may be located in the middle of the open ring at a position substantially opposite of the light pipes 325.

This arrangement permits that the switch arm 326 may be received in the groove 25 when the switch arm 326 is deflected in a state in which the module 11 is mounted onto the button 12 of the drug delivery device 1. In this state, the free end 327 extends through a further aperture 20 in the button 12 into the drug delivery device 1. Thus, the free end 327 and the switch arm 326 may be deflected if components within the drug delivery device 1 move relative to the button 12. More specifically, at the beginning of dose dispensing, a user presses on the proximal end of the modue 11, thereby displacing the module 11 with the button 12 relative to e.g. the number sleeve 23, or alternatively relative to the drive sleeve. This results in the free end 327 contacting e.g. the number sleeve 23 and deflecting the switch arm 326 which in turn actuates switch 331 on the distal side of the PCBA 330 which may trigger waking up of the module 11.

The PCBA 330 may comprise or form the processor 110, the sensor arrangement 120, the communication unit 130, the electronic user feedback generator 140 and the memory 150. The PCBA 330 is supported on the component 320 which acts as a chassis in the module 11. In addition to the LEDs 121 and photo detectors 122, one or more LED(s) 141 may be provided on the PCBA 330. In addition, a switch 331 may be provided on the PCBA 330, e.g. on a distal side facing towards the drug delivery device 1.

Figures 6a, 6b and 6c show three similar embodients of the design of the button 12 of the drug delivery device 1. In Figure 6a, the button 12 comprises the groove 25 with apertures 19, 20 for the light pipes 325 and the free end 327 of the switch arm 326, respectively. Further, recesses 22 are provided for snap engagement with the attachment elements 324 of the module 11. In Figures 6b and 6c, an additional inner grove is provided in which the module lockout forms 323 are arranged. Apertures 19 and 20 are formed as one common, slot-like opening in Figure 6c instead of separate openings as in Figure 6a.

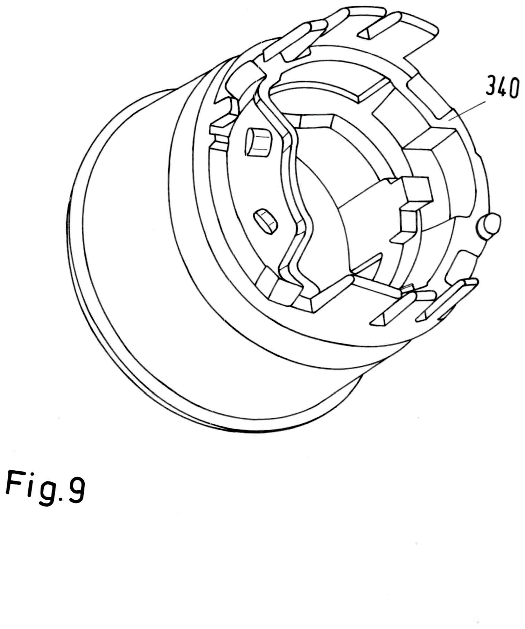

As depicted in Figure 9, a potting compound 340 or filling layer may be applied preventing ingress of dust and water to the conductive areas of the PCBA 330. In addition or as an alternative, only one side or both sides of the PCBA 330 may be covered at least partially or at all locations that are not covered by electronic parts by a potting material or by a potting compound or by a conformal coating layer. For example, the chassis component 320 is configured to separate the potting compound from an electrical sensor and/or from a radiation source of the detector unit.

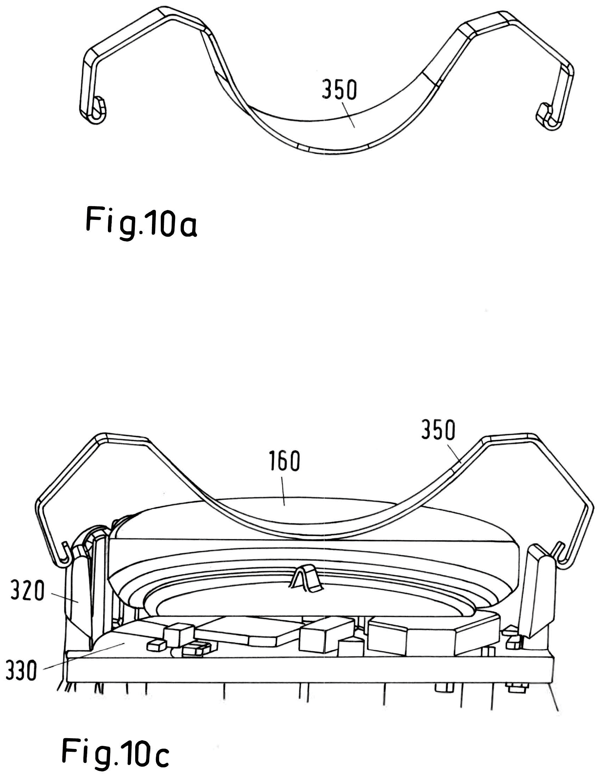

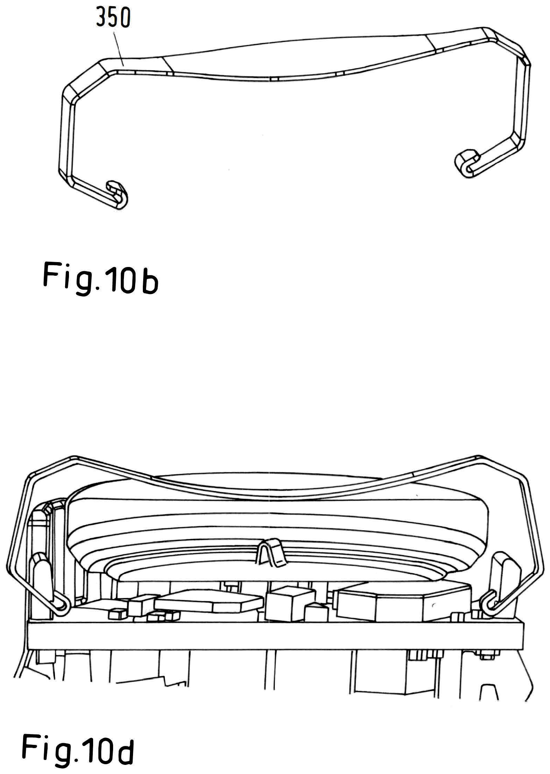

Figures 10a to 10d show an exemplary use of a power source clip 350 which may be attached to the chassis component 320 in order to retain the the coin cell 160 on the cassis component 320 and to connect the coin cell 160 with the PCBA 330. The clip 350 has a curved form in its unbiased state as depicted in Figures 10a and 10c. In contrast to that, the clip 350 has a flater curvature in a configuration mounted on the chassis component 320 (see Figures 10b and 10d). The chassis component 320 may have corresponding snap features for attachment of clip 350, in particular of the free ends of clip 350.

The clip 350 consists of an elastiacally deformable and electrically conductive material, e.g. a metal. A central portion of the clip 350 is adapted to contact one terminal of coin cell 160, the upper terminal in Figures 10c and 10d, whereas at least one of the free ends of the clip 350 is adapted to contact a respective terminal on the PCBA 330 if the clip 350 is attached to the chassis component 320 (Figure 10d). For this purpose, at least a portion of the clip 350, e.g. its free ends as shown in Figure 10d, may extend through a respective aperture in the chassis component 320.

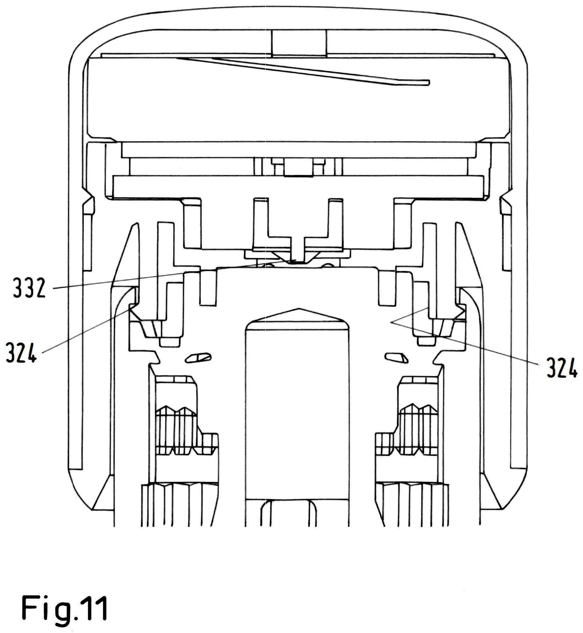

Still further, Figure 11 depicts an alternative embodiment with an additional switch 332 provided on the PCBA 330. This switch 332 is actuated if the module 11 is fully and correctly fitted onto the button 12 by contact between the distal switch surface and a proximally facing button surface. Such a switch may be used to activate the processor 110 or components thereof, e.g. from a no-power or sleeping mode of the module 11 when the module is not attached to the device 1.

Although described mainly with respect to a drug delivery device1 having a similar working principle as the device disclosed in EP 2 890 435, the electronic module 11 is applicable to any other type of drug delivery device having component parts performing a relative axial and/or rotational movement in defined conditions or states.

### Reference Numerals

| | | | |
|---|---|---|---|
| 1 | device | | |
| 10 | housing | 150 | memory |
| 11 | button module | | |
| 12 | dial grip/button | 160 | power source (coin cell) |
| 13 | dosage window | | |
| 14 | container/container receptacle | 200 | smartphone (other device) |
| 15 | needle | | |
| 16 | inner needle cap | 310 | cap |
| 17 | outer needle cap | | |
| 18 | cap | 320 | (chassis) component |
| | | 321 | skirt |
| 19 | aperture | 322 | user feedback surface |
| 20 | aperture | 323 | module lockout form |
| 21 | device lockout form | 324 | attachment element |
| 22 | recess | 325 | light pipe |
| 23 | nuber sleeve | 326 | switch arm |
| 24 | teeth | 327 | free end |
| 25 | groove | 328 | rim |
| | | 329 | collar portion |
| 110 | processor | 330 | PCBA |
| 120 | sensor arrangement | 331 | switch |
| 121 | LED | 332 | switch |
| 122 | photo detector | 340 | potting compound |
| 130 | communication unit | 350 | power source clip |
| 140 | electronic user feedback generator | | |
| 141 | LED | | |

## Claims

1. An electronic module (11) for releasable attachment to a drug delivery device (1), the module (11) comprising a printed circuit board assembly (330), a power source (160) and a component (320), wherein the power source (160) is a coin cell, and wherein the component (320) which is a chassis of the module (11) comprises the following feature:
- at least one attachment element (324) for releasable attachment of the electronic module (11) on the drug delivery device (1),
**characterized in that** the coin cell is secured and connected to the printed circuit board assembly (330) by means of a power source clip (350) which is attached to the chassis component.

2. The electronic module according to claim 1, wherein the power source clip (350) consists of an elastically deformable and electrically conductive material.

3. The electronic module according to claim 1 or 2, wherein the power source clip comprises free ends and a central portion, and wherein the central portion of the power source clip (350) is adapted to contact an upper terminal of the coin cell and the free ends of the power source clip (350) are adapted to contact a respective terminal on the printed circuit board assembly (330).

4. The electronic module according to claim 3, wherein the free ends of the power source clip (350) extend through a respective aperture in the chassis component.

5. The electronic module (11) according to claim 1, wherein the component (320) further comprises at least one of the following features:
- at least one module lockout form (323) adapted for mating abutment with a corresponding device lockout form (21) of a dedicated drug delivery device (1),
- at least one light pipe (325) for guiding a light beam from a light source (121) to a reflective surface (24) of the drug delivery device (1) and from said reflective surface (24) to a light detector sensor (122),
- at least one elastically deformable switch arm (326, 327),
- the component (320) is a unitary component part injection molded from a thermoplastic material, which is highly transparent to light.

6. The electronic module according to claim 1 further comprising a cap (310), wherein the component (320) is rigidly attached to the cap (310) and supports the printed circuit board assembly (330) within the cap (310).

7. The electronic module according to claim 5, wherein the at least one module lockout form (323) comprises a profiled protrusion or seat adapted for mating abutment with a corresponding contoured seat or protrusion of the device lockout form (21) of said dedicated drug delivery device (1).

8. The electronic module according to claim 5 to 7, wherein the at least one module lockout form (323) prevents attachment of the module (11) on a non-mating drug delivery device (1).

9. The electronic module according to any of the preceding claims, wherein the at least one attachment element (324) for releasable attachment of the module (11) on a drug delivery device (1) comprises at least one elastically deformable arm with a snap protrusion or snap recess for releasable engagement with a corresponding snap recess (22) or snap protrusion of the drug delivery device (1).

10. The electronic module according to claim 5 to 9, wherein the at least one light pipe (325) is an elongate cuboid shaped protrusion or a protrusion in the shape of a conical frustum having two opposite end faces and at least one sidewall, wherein at least one of the two opposite end faces have a surface roughness higher than the surface roughness of the at least one sidewall.

11. The electronic module according to claim 5 to 10, comprising at least two light pipes (325) protruding axially in the same direction from an inner portion (328) of the component (320).

12. The electronic module according to claims 5 to 11, wherein the at least one elastically deformable switch arm (326) comprises a free end (327) and is deflectable with respect to the component (320) in order to actuate an electronic switch (331).

13. The electronic module according to claim 12, wherein the electronic switch (331) is mounted on a distal side of the printed circuit board assembly (330).

14. The electronic module according to claim 12 or 13, wherein the free end (327) of the at least one elastically deformable switch arm (326) is configured to enter through an aperture (20) in a button (12) of the drug delivery device (1) into the drug delivery device (1).

15. The electronic module according to any of claims 6 to 11, wherein the module (11) comprises a user feedback surface (322), wherein the user feed back surface (322) is encased by the cap (310), and wherein the printed circuit board assembly (330) and the power source (160) are interposed between the cap (310) and the component (320).

16. The electronic module according to any of the preceding claims, further comprising:
- at least one processor (110),
- a sensor arrangement (120) connected to the at least one processor (110) and operable to generate measurement data indicative of a dose setting operation and/or a dose delivery operation of the dedicated drug delivery device (1),
- a communication unit (130) with a wireless communication interface connected to the at least one processor (110) and operable to establish communication with another device (200) and to transfer data to said other device (200),
- at least one electronic user feedback generator (140) connected to the at least one processor (110) and operable to generate a feedback signal, and
- a memory (150) for storing measurement data.

17. The electronic module according to any of the preceding claims, wherein the electronic module (11) comprises a clip feature for forming a clip connection with the drug delivery device (1) comprising a complementary-shaped counter fastening element.

18. The electronic module according to claim 17, wherein the electronic module (11) comprises two clip features as attachment elements (324) that protrude distally from an internal rim (328) of the chassis component (320), wherein the clip features axially retain and rotationally orientate the electronic module (11) on the drug delivery device (1).

19. The electronic module according to claims 5 to 18, wherein the at least one light pipe (325) is a protrusion in the form of a truncated pyramid or a cylindrical shape.

20. The electronic module according to any of claims 5 to 19, wherein the at least one light pipe (325) extends axially over the at least one attachment element (324).

21. The electronic module according to any of claims 5 to 20, wherein the at least one light pipe (325) extends axially over the at least one module lockout form (323).

22. A drug delivery device (1) for delivery of a medicament, the drug delivery device (1) comprising a dose setting and drive mechanism (23), which is configured to perform a dose setting operation for setting a dose to be delivered by the drug delivery device (1) and a dose delivery operation for delivering the set dose and which comprises a first member (20), and a container receptacle (14) which is permanently or releasably connected to the dose setting and drive mechanism and which is adapted to receive a container containing a medicament, **characterized in that** the drug delivery device (1) comprises the electronic module (11) according to any one of the preceding claims.
